# EUROPEAN PATENT APPLICATION

(11) **EP 1 334 707 A1**
(43) Date of publication of application: **13.08.2003**
(21) Application number: 03008928.8
(22) Date of filing: 20.08.1997
(51) Int. Cl.: A61F 7/00

(54) **Applying thermal therapy**

(30) Priority: 20.08.1996 US 700290
(62) Divisional of application: 97936516.0
(71) Applicant: Kolen, Paul T., Encinitas, CA 92024 (US); Ford, Thomas D., San Diego, CA 92124 (US)
(72) Inventor: Kolen, Paul T., Encinitas, CA 92024 (US); Ford, Thomas D., San Diego, CA 92124 (US)
(74) Representative: Cabinet Hirsch

(57) **Abstract**

This invention concerns a thermal therapy device for applying thermal therapy to a therapy site on a mammalian body, said device comprising a thermal pad for applying thermal therapy to the therapy site, said thermal pad comprising a plurality of vents for providing air access through said thermal pad when said thermal pad is held in place at the therapy site.

## Description

### Background of the Invention

This invention relates to devices and methods for applying thermal therapy to living tissue.

Thermal therapy involves the application of heat or cold to tissue to heal and rehabilitate injuries, such as bruises, sprains, or other trauma to bone, muscle, ligaments, tendons, and skin. Cold therapy can be used to reduce swelling, reduce pain and promote healing of injured tissue. Heat therapy can be used to loosen joint tissue, such as ligaments and tendons, to increase range of motion, e.g., before strenuous activity. Thermal therapy can be used after surgery to reduce pain and swelling and promote healing. Thermal therapy can also be used as part of an orthopedic therapy program, a sports medicine program, and to heal and rehabilitate animals, such as thoroughbred race horses.

### Summary of the Invention

In one aspect, the invention features novel thermal therapy schemes (devices and methods) for applying thermal therapy to various therapy sites on a mammalian body. The novel schemes generally comprise a thermal pad for applying thermal therapy to the therapy site, and a retainer integrally receiving the thermal pad to form a single unit and retaining the thermal pad against the therapy site with compressive force to hold the thermal pad in place in uniform thermal contact with the therapy site.

In another aspect, the invention features a thermal therapy device comprising a thermal pad for applying thermal therapy to a therapy site, the thermal pad comprising a plurality of vents for providing air access through the thermal pad when the thermal pad is held in place at the therapy site.

In yet another aspect, the invention features a thermal therapy device comprising: a thermal pad for applying thermal therapy to a therapy site; a recirculating fluid loop comprising a fluid channel defined by the thermal pad; a pump for circulating fluid through the recirculating fluid loop; a thermal reservoir; a heat exchanger coupling the thermal reservoir with the recirculating fluid loop; and a hand-held controller selectively connectable to the heat exchanger for enabling adjustable control of therapy temperature.

The invention also features a thermal therapy device comprising: a thermal therapy pad for applying thermal therapy to a therapy site; a recirculating fluid loop comprising a fluid channel defined by the thermal pad; a thermal reservoir; a heat exchanger coupling the thermal reservoir with the recirculating fluid loop, the heat exchanger being constructed and arranged to selectively mix fluid recirculating in the fluid loop with fluid from the thermal reservoir in an adjustable mixing ratio to achieve the selected therapy temperature at the therapy site; and a hand-held controller selectively connectable to the heat exchanger for enabling adjustable control of therapy temperature.

In another aspect, the invention features a thermal therapy device comprising: a thermal pad for applying thermal therapy to the therapy site; a recirculating fluid loop comprising a fluid channel defined by the thermal pad; a pump for circulating fluid through the recirculating fluid loop; a thermal reservoir; and a second fluid loop thermally coupling the recirculating fluid loop to the thermal reservoir and allowing fluid exchange therebetween. The thermal therapy device preferably includes a flow controller for adjusting the amount of fluid exchange between the recirculating fluid loop and the reservoir.

Features and advantages of the invention will become apparent from the following.

### Brief Description of the Drawings

Fig. 1 is a diagrammatic view of a thermal therapy device of the invention for applying thermal therapy to the knee of a person.
Fig. 2 is a perspective view, partially broken away, of a reservoir housing for a thermal therapy device of the invention. Fig. 2A is a schematic diagram of the thermal therapy device of Fig. 1, including a thermal reservoir, a heat exchanger, and a treatment pad.
Fig. 3 is a diagrammatic view of insulated fluid supply and return lines a thermal therapy device of the invention.
Fig. 4 is a diagrammatic view of a thermal pad.
Figs 5 and 5A are cross-sectional views of the thermal pad of Fig. 4 shown in inflated condition and deflated condition, respectively.
Fig. 6 is a therapy temperature time profile programmed into a therapy device controller.
Fig. 7 is a schematic diagram of a thermal therapy device.
Fig. 8 is a schematic diagram of another alternative thermal therapy device.
Figs. 9-9B are schematic diagrams of thermal therapy devices using a single thermal reservoir.
Figs. 10 and 10A are diagrammatic front and side views of a thermal therapy device. Fig. 10B is a diagrammatic view of two thermal pads for use with the thermal therapy device of Figs. 10 and 10A. Fig. 10C is a diagrammatic view, shown in partial cross-section, of a vent in one of the thermal pads shown in Fig. 10B.
Fig. 11 is a diagrammatic view of thermal therapy device.
Fig. 12 is an exploded diagrammatic view of a thermal therapy device.
Fig. 13 is a diagrammatic view of a thermal therapy device.
Fig. 14 is a diagrammatic view of a portable reservoir, a hand-held controller, and a thermometer of a thermal therapy device.

### Description of Preferred Embodiments

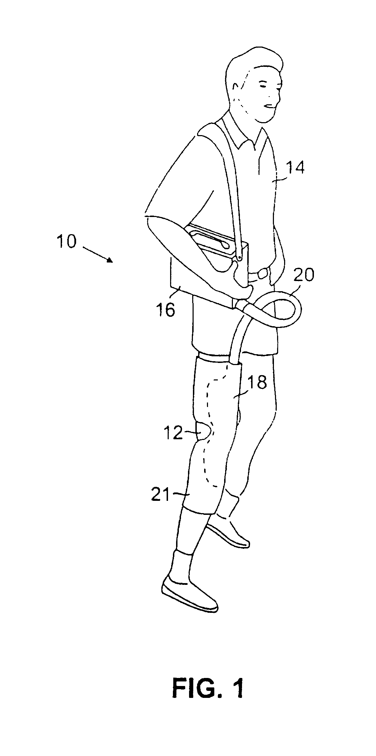

Referring to Fig. 1, a thermal therapy device 10 applies temperature-controlled thermal therapy to the knee 12 of a person 14. Thermal therapy device 10 includes a portable reservoir 16 that is connected to a thermal pad 18 by a thermally insulated supply and return assembly 20. As described in detail below, thermal therapy device 10 uniformly heats or cools the person's knee according to a predetermined temperature schedule, and can be programmed to stimulate the patient's knee by controllably varying the inflation pressure inside thermal pad 18. A wrap 21, which is made of, e.g., neoprene rubber, is shaped to snugly hold thermal pad 18 in place at the therapy site, while allowing the thermal pad to expand and contract during tactile stimulation of the person's knee. Wrap 21 is held in place by VELCRO®, i.e., hook-and-loop type, fasteners that allow the wrap to be selectively adjusted to fit firmly, evenly and comfortably in place at the therapy site.

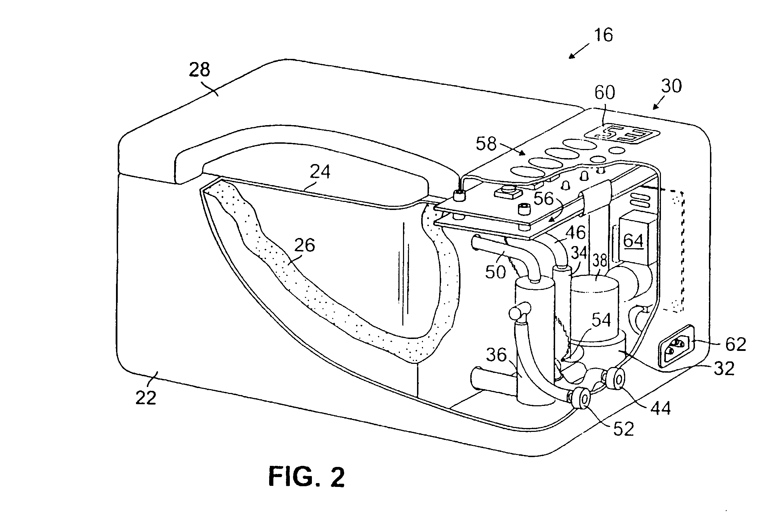

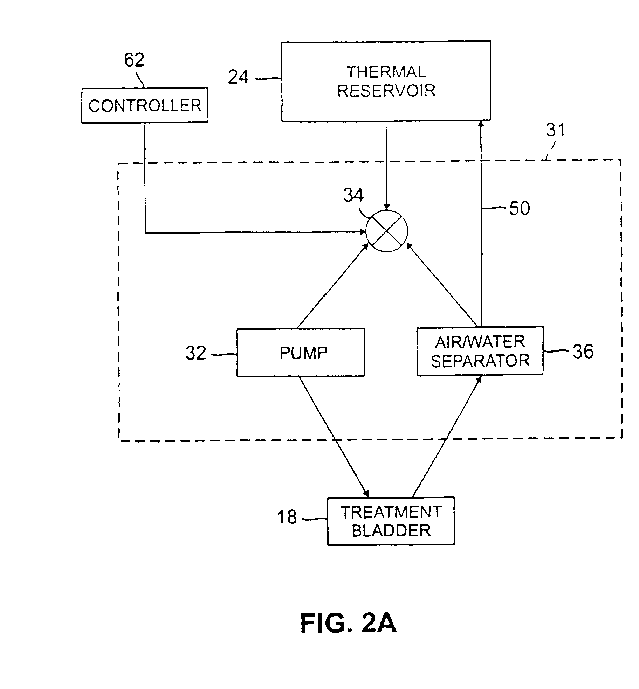

Referring to Fig. 2, in one embodiment, portable reservoir 16 includes a protective outer case 22 and an inner, leak-proof thermal reservoir 24. Reservoir 24 is thermally insulated by a thermal lining 26, which fills the space between the outer walls of reservoir 24 and the inner wall of outer case 22. A thermally insulated lid 28 opens to provide access to reservoir 24. Lid 28 includes a seal (not shown) sized and constructed to form a fluid-tight seal between lid 28 and the top opening of reservoir 24 when the lid is closed, to prevent fluid from leaking during use.

A fluid control system 30 is contained within the housing, in a compartment space provided between the thermal reservoir and the outer casing. A heat exchanger 31, within the fluid control system, includes a pump 32, a single pole, double-throw priming valve 34, and an air/water separator 36. (In some embodiments, a solenoid valve may replace the single pole, double-throw priming valve.) Pump 32 includes an input 40 and an output 42, and is powered by a motor 38. Pump input 40 is connected to the output of priming valve 34, and pump output 42 is connected to a quick-disconnect outlet 44, through which fluid flows from the pump to the thermal pad. An input 46 of priming valve 34 is connected to thermal reservoir 24, and an input 48 of the priming valve is connected to an output of air/water separator 36. An overflow tube 50 provides a fluid path between the air/water separator and thermal reservoir 24. Air/water separator 36 receives fluid from the thermal pad through tubing 51 from a quick-disconnect inlet 52. The temperature of the fluid that is supplied to the thermal pad is monitored by thermistors 54 placed in the fluid paths of the supply and return lines of supply and return assembly 20.

Reservoir 24 accommodates crushed ice, ice cubes and pre-formed freezable cold sources, such as, those commonly used in portable coolers. The reservoir is easily recharged with additional ice if needed during use, without requiring the person to remove the thermal pad from the therapy site. For heat therapy, hot water can be introduced into the reservoir, or the reservoir fluid can be controllably heated using an immersion heater. The temperature of the fluid supplied to the thermal pad is controlled by a microprocessor-based controller 56 (control electronics). Based on the therapy temperature measured by thermistors 54, controller 56 produces an audible alarm signal when the cold or heat source in the reservoir is exhausted and the desired therapy temperature cannot be maintained within preset tolerances; an alarm also sounds if the unit detects a restricted flow in the circulation system. Controller 56 incorporates a non-volatile electronic memory for storing, recalling and implementing one or more preprogrammed or user-defined therapy temperature time profiles. Input keys 58 are used to program the desired temperature profile into controller memory. The monitored temperature is shown on a digital display 60. Display 60 may also indicate the amount of therapy time remaining. Electrical power is supplied to fluid control system 30 from a conventional wall outlet, through power connector 62, to switching power electronics 64.

As shown in Fig. 2A, heat exchanger 31 controls the temperature of thermal pad 18 by mixing a controlled amount of fluid from thermal reservoir 24 with recirculated fluid returning from the thermal pad through air/water separator 36. By using the real-time temperature information generated by thermistors 54, controller 56 adjusts valve 34 to control the proportion of reservoir fluid that mixes with the recirculation fluid received from the thermal pad to achieve the prescribed treatment pad temperature. For example, if the fluid injected to the thermal pad through quick-disconnect outlet 44 is at the prescribed temperature, controller 56 will adjust valve 34 so that no fluid is received from reservoir 24; in other words, pump 32, thermal pad 18, and air/water separator 36 form a closed-loop system (the fluid may flow in either direction). If the output fluid temperature varies from the desired set-point, however, controller 56 will adjust valve 34 so that fluid from reservoir 24 mixes with recirculated water from air/water separator 36 in the proportion selected to achieve the desired output fluid temperature. Because the fluid volume in the fluid flow path defined by the pump, the thermal pad, and the air/water separator is substantially fixed, some recirculated fluid will be displaced and flow into reservoir 24 via overflow tube 50 to complete the heat exchange process.

To ensure uniform temperature distribution at the therapy site (or sites), a high flow rate is used to reduce the temperature gradient that develops across the thermal pad as a result of heat transfer at the treatment site. In some embodiments, the thermal therapy device includes multiple thermal pads coupled in series, which can be used, e.g., in the treatment of post bilateral surgery therapy. High flow rates are generally needed in these multi-pad embodiments to reduce the temperature differential between the upstream and downstream thermal pads. The flow rate can also be selected based on the anticipated heat load at the treatment site.

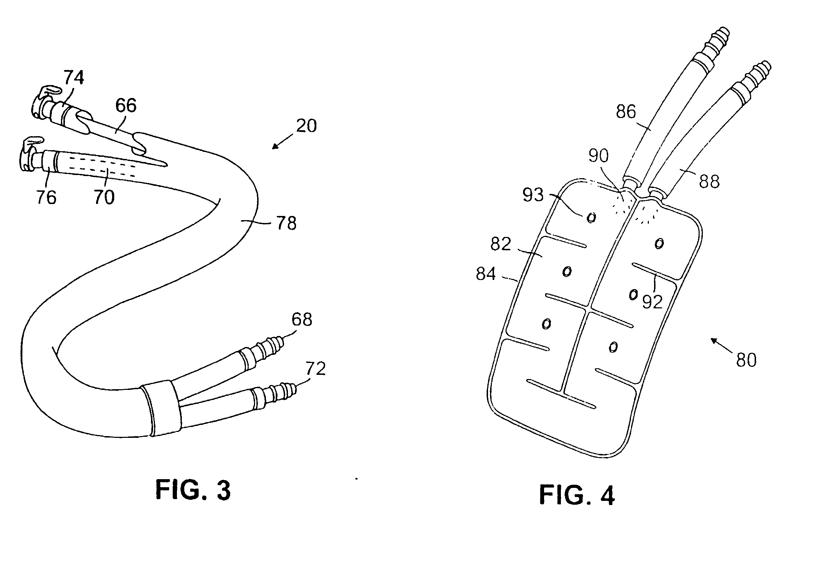

Referring to Fig. 3, insulated supply and return assembly 20 includes a flexible fluid supply line 66 that connects to quick-disconnect outlet 44 via a mating quick-disconnect connector 68, and a flexible fluid return line 70 that connects to quick-disconnect return inlet 52. In the embodiment shown in Fig. 3, the supply and return line assembly 20 is attached to the thermal pad via quick-disconnect connectors 74, 76. The flexible supply and return lines 66, 70 are encased in thermal insulation 78 (e.g., polyurethane foam) that reduces ambient heat loads, makes the entire line assembly fluid tight, durable and flexible, and is more comfortable for the user to handle. The length of the supply and return line assembly is selected based at least in part on the size of the user and on the anticipated thermal therapy treatment.

Various thermal pad shapes and sizes are contemplated depending on the selected treatment site (e.g., ankle, knee, elbow), with the object being to sufficiently cover the treatment site to achieve optimal therapy results. In the embodiment shown in Fig. 4, a thermal pad 80 is formed of two layers of flexible polymeric material 82 that are heat-welded or otherwise sealed together at the outer edge 84 of the thermal pad. In this embodiment, supply and return lines 86, 88 are permanently attached to thermal pad 80 by a leak proof seal 90. Thermal pad 80 also includes one or more internal seams 92 (or internal walls) and "dimple" welds 93, which direct the flow of cooling fluid uniformly through the thermal pad; the internal seams and dimple welds also control the expansion and contraction of the thermal pad.

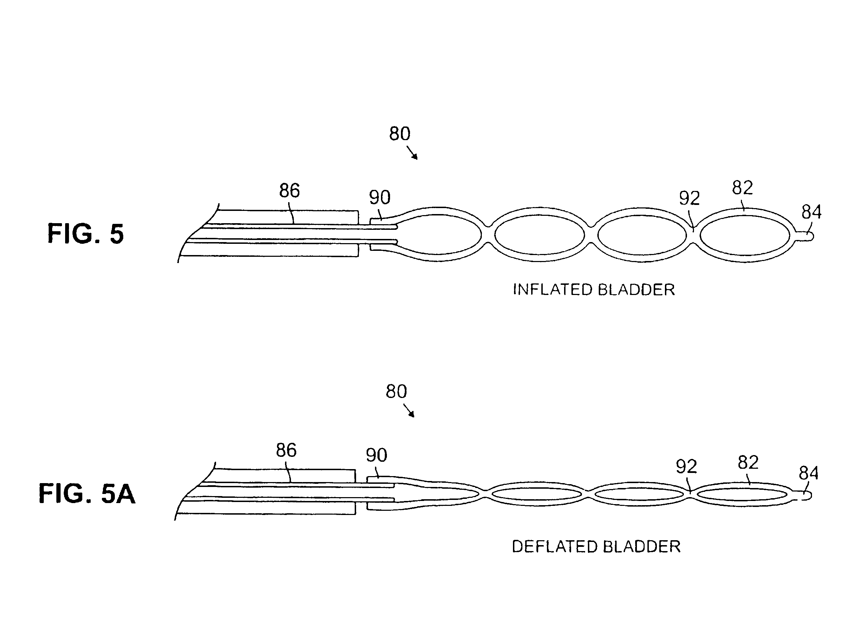

Referring to Figs. 5 and 5A, thermal pad 80 is constructed to allow the thermal pad to expand (Fig. 5) and to contract (Fig. 5A) in response to varying fluid pressures applied by the heat exchanger, and to ensure a uniform distribution of circulating fluid within the thermal pad. To achieve tactile stimulation, pump 32 is turned off and on at preprogrammed intervals to periodically allow the pressure in the thermal pad to be cycled between low and high values. Such a periodic pressure variation in the thermal pad provides tactile stimulation at the therapy site while achieving the desired therapy temperature. Controller 56 (Fig. 2) can be programmed to simultaneously provide the desired temperature profile and the desired tactile stimulation.

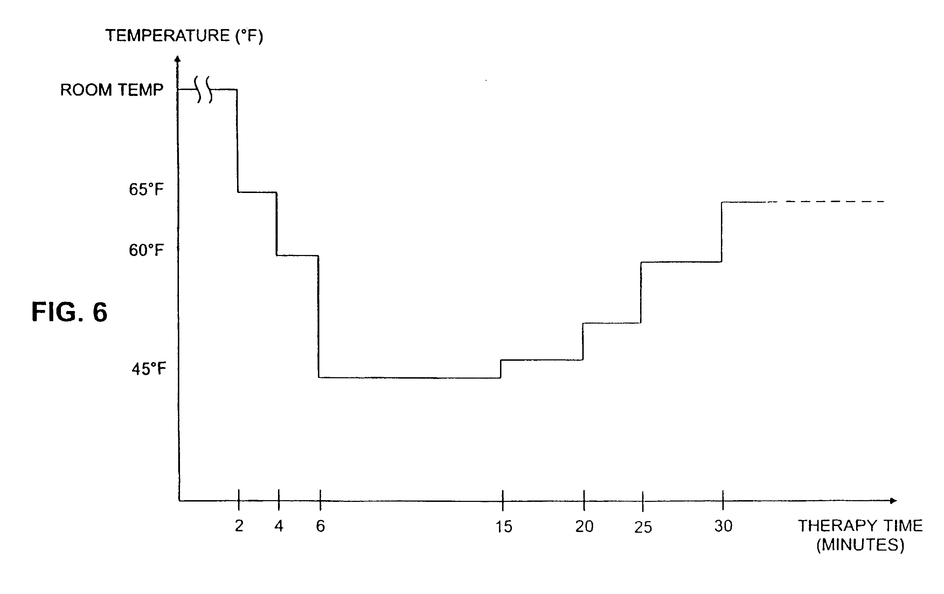

Although a constant therapy temperature time profile may be programmed into controller 56, it is preferable to vary the temperature during treatment to avoid discomfort and permit long term thermal therapy without causing tissue damage. As shown in Fig. 6, a preferred cold therapy temperature time profile calls for a reduction in the applied therapy temperature from room temperature to a predetermined minimum temperature (e.g., 45° F) during an initial treatment stage; during an intermediate stage the minimum therapy temperature is maintained for a fixed period (e.g., nine minutes); and during a final treatment stage the temperature is increased at regular intervals (e.g., every five minutes) until the treatment temperature is at about 65° F. The applied therapy temperature is maintained at 65° F for the duration of the prescribed treatment period.

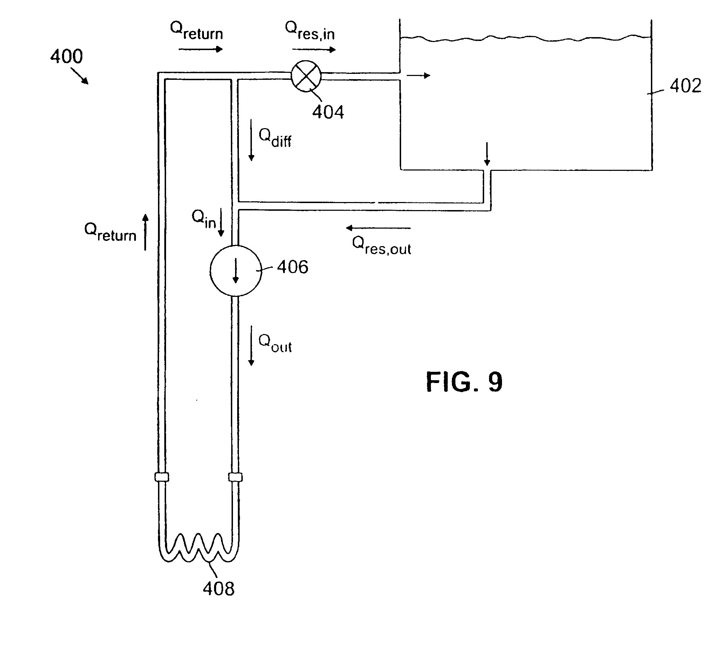

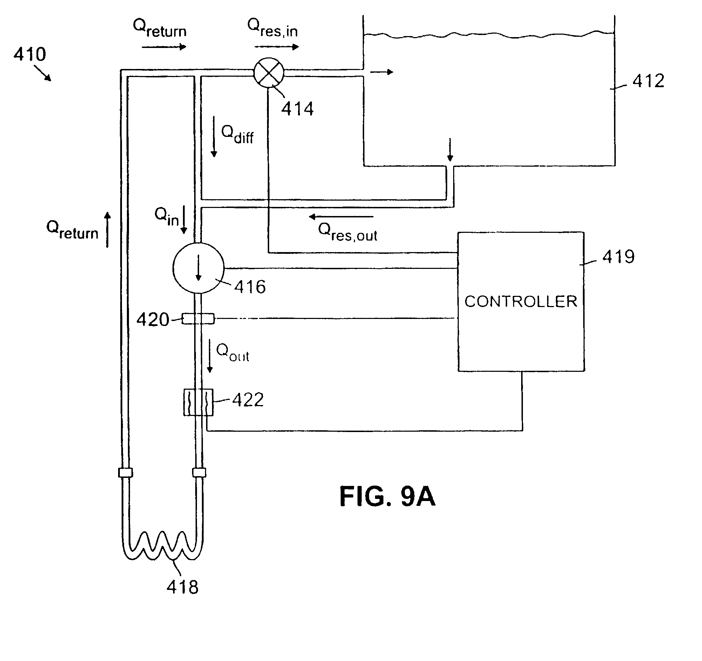

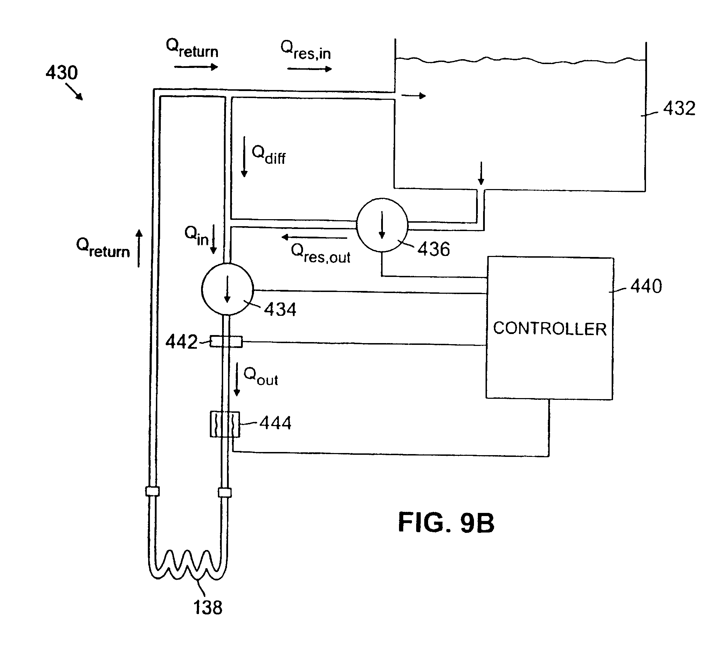

Other embodiments are within the scope of the claims. For example, in one embodiment, the thermal therapy device uses two pumps, instead of the combination of a single pump and a single pole, double-throw valve, to achieve closed-loop temperature control. Closed-loop temperature control can be achieved using simple analog control electronics, such as a solid state thermostat with the therapy site temperature selected with a mechanically operated device, such as a potentiometer in conjunction with a temperature read-out device. An in-line flow heater can be placed in the circulation loop in one or more of the above embodiments to allow the fluid within the circulation loop to be readily heated to a desired temperature (see, e.g., the embodiments of Figs. 9A and 9B, below).

### Automatic Closed-Loop Heat Exchanger

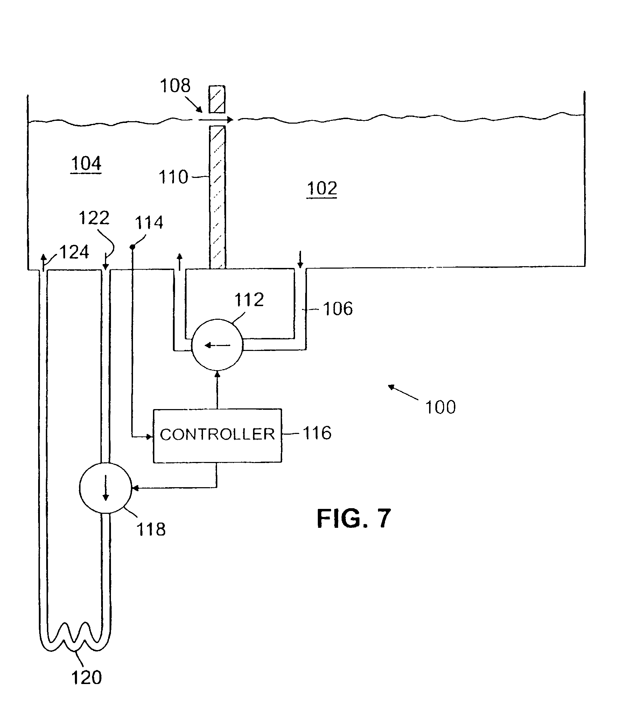

Referring to Fig. 7, a thermal therapy device 100 includes a primary reservoir 102 that is coupled to a secondary reservoir 104 by an active flow path 106 and by an overflow path 108; the primary and secondary reservoirs are separated by a thermal barrier 110. Active flow path 106 includes a dedicated constant pressure circulation pump 112. The temperature of the fluid in the secondary reservoir is monitored using a thermal sensor 114. A controller 116 adjusts the pump rate to maintain the temperature in the secondary reservoir at a desired temperature by controlling the fluid flow from the primary reservoir. Any excess fluid in the secondary reservoir is returned to the primary reservoir through overflow fluid path 108, completing the heat exchange circuit. A high speed circulation pump 118 controllably injects fluid from secondary reservoir 104 into a thermal therapy thermal pad 120.

The closed-loop electronic control allows the temperature of the secondary reservoir fluid to be maintained at a desired set-point value within about ±0.3° F in a desired temperature range (e.g., 40° F to 70° F, with the temperature of the primary reservoir fluid at about 35° F) for practical thermal loads. When the secondary reservoir fluid temperature corresponds to the programmed temperature, the controller adjusts the fluid transfer between the primary reservoir and the circulation loop so that the thermal transfer from the primary reservoir makes up for the thermal transfer at the treatment site through the thermal pad, which is represented by the difference in temperature between fluid 122 injected into the thermal pad and fluid 124 returning from the thermal pad. When the temperature profile programmed into controller 116 indicates that the therapy temperature is to be changed, the controller increases or decreases the fluid transfer, depending on whether the applied temperature is to be increased or decreased. Because the fluid from the secondary reservoir is injected into the thermal pad at a high rate, the thermal load at the treatment site does not substantially affect the temperature of the fluid through the thermal pad and, consequently, the temperature differential across the thermal pad is maintained within about 2-3° F of the programmed set-point value.

Reservoir 102 accommodates crushed ice, ice cubes and pre-formed freezable cold sources, such as, those commonly used in portable coolers. The reservoir is easily recharged with additional ice if needed during use, without requiring the person to remove the thermal pad from the therapy site. For heat therapy, hot water can be introduced into the reservoir, or the reservoir fluid can be controllably heated using an immersion heater. Because ice water is used for the primary thermal reservoir, the thermal therapy device is highly cost-effective.

### Manual Open-Loop Heat Exchanger

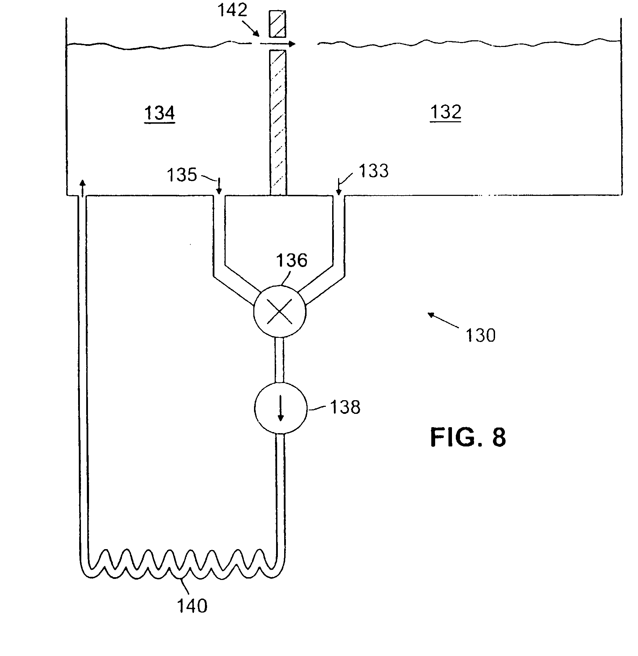

Referring to Fig. 8, a thermal therapy device 130 includes a primary thermal reservoir 132 and a secondary thermal reservoir 134. As in the embodiments described above, primary thermal reservoir 132 can accommodate a mixture of water and crushed ice or other cold source, or heated fluid. The mixing ratio of primary reservoir fluid 133 and recirculation fluid 135 from the secondary reservoir is adjusted by a manually-controlled valve 136. A high speed circulation pump 138 controls the flow of fluid into a thermal pad 140. Any excess fluid in the secondary reservoir is returned to the primary reservoir through overflow path 142, which completes the heat exchange circuit.

By adjusting valve 136 a user can empirically control the temperature of the secondary reservoir in an open-loop fashion. The water is mixed within the pump to create a near constant temperature within the circulation loop and the secondary reservoir. Preferably, valve 136 includes markings that indicate the correspondence between valve position and treatment temperature. For example, in one embodiment, valve 136 includes a marking that corresponds to a mixing ratio needed to provide a temperature of 45° F, which the user may apply to the treatment site for the initial period of treatment (e.g., ten to fifteen minutes). Valve 136 also includes a second marking that corresponds to a mixing ratio needed to provide a temperature of 65° F, which the user may apply to the treatment site indefinitely.

### Other Single Reservoir Embodiments

Temperature-controlled fluid from the primary reservoir can be injected directly into the circulation loop without loss of temperature control performance, allowing the secondary reservoir to be omitted. For example, referring to Fig. 9, an open-loop, manually-actuated thermal therapy device 400 includes a thermal reservoir 402, an adjustable manual valve 404, a circulation pump 406, and a thermal pad 408. Because the fluid from the thermal reservoir is substantially incompressible, Qₒᵤₜ=Qᵢₙ and Q_{diff}=Qᵣₑₜᵤᵣₙ - Q_{res,in}, where Q_{res,in} is controlled by the valve position which allows the user to adjust the amount of Qᵣₑₜᵤᵣₙ that returns to the inlet of circulation pump 406. Since Qᵢₙ=Q_{diff} + Q_{res,out} and Qᵢₙ=Qₒᵤₜ, Qₒᵤₜ=Q_{diff} + Q_{res,out}; and since Q_{res,out}=Q_{res,in}, Qᵣₑₜᵤᵣₙ=Q_{diff} and Q_{res,out}=0 when the valve is closed (Q_{res,in}=0). Therefore, the valve position and the corresponding flow (Q_{res,in}) controls the fluid temperature in the Qₒᵤₜ/Qᵣₑₜᵤᵣₙ circulation loop, where the heat absorbed by the Qₒᵤₜ/Qᵣₑₜᵤᵣₙ loop is equal to the net heat transferred to the thermal reservoir via Q_{res,out} and Q_{res,in}.

Referring to Fig. 9A, a closed-loop thermal therapy device 410 includes a thermal reservoir 412, a normally closed solenoid valve 414, a circulation pump 416, and a thermal pad 418. An electronic controller 419 senses fluid temperature in the Qₒᵤₜ/Qᵣₑₜᵤᵣₙ circulation loop using a thermal sensor 420, and controls the valve position based on the sensed temperature. If the fluid temperature is within a specified range, controller 419 closes valve 414, resulting in Q_{res,in}=Q_{res,out}=0. If the temperature increases above the set-point temperature, controller 419 opens valve 414 to inject cooler fluid from reservoir 412 into the circulation loop (Q_{res,out}) to complete the control loop. An in-line flow heater 422 allows fluid in the circulation loop to be readily heated without requiring replacement of cold fluid in reservoir 412 with heated fluid. When valve 414 is closed, there is no fluid exchange between the circulation loop and the reservoir; this allows the relatively small volume of water in the circulation loop to be readily heated to a desired temperature with heater 422.

Referring to Fig. 9B, a closed-loop thermal therapy device 430 includes a thermal reservoir 432, a circulation pump 434, a transfer pump 436, and a thermal pad 438. An electronic controller 440 senses fluid temperature in the Qₒᵤₜ/Qᵣₑₜᵤᵣₙ circulation loop using a thermal sensor 442, and controls the fluid transfer through transfer pump 436 based on the sensed temperature. The amount of fluid injected into the inlet of the transfer pump can vary continuously (using, e.g., PID feedback control) or in an on/off fashion using, e.g., a thermostat controller. If the fluid temperature is within a specified range, controller 440 turns off the transfer pump, resulting in Q_{res,in}=Q_{res,out}=0. If the temperature increases above the set-point temperature, controller 440 turns on transfer pump 436 to inject cooler fluid from reservoir 432 into the circulation loop (Q_{res,out}) to complete the control loop. An in-line flow heater 444 allows the fluid in the circulation loop to be readily heated without requiring replacement of cold fluid in reservoir 432 with heated fluid. When transfer pump 436 is turned off, there is no fluid exchange between the circulation loop and the reservoir; this allows the small volume of water in the circulation loop to be readily heated to a desired temperature with heater 444.

In the embodiment of Fig. 9B, Qₒᵤₜ=Qᵢₙ, Q_{diff}=Qᵣₑₜᵤᵣₙ - Q_{res,in}, and Qᵢₙ=Q_{diff} + Q_{res,out}. In this configuration, Q_{res,out} is actively controlled and Q_{res,in} is passively controlled. If the transfer pump is off (unpressurized), no fluid is forced into the inlet side of the transfer pump (Q_{res,out}=0), resulting in Qᵢₙ=Q_{diff}. Since Qᵢₙ=Qₒᵤₜ=Qᵣₑₜᵤᵣₙ, Qᵣₑₜᵤᵣₙ=Q_{diff}; in which case Q_{res,in}=0. In other words, if no reservoir fluid is forced into the inlet side of the transfer pump, no return fluid is discharged back into the reservoir, resulting in no heat transfer between the reservoir and the circulation loop.

Still other embodiments are within the scope of the claims. For example, in the following embodiments, described in the context of specific thermal therapy procedures, retaining structures are constructed to integrally receive one or more thermal pads and comfortably retain the pads in place at therapy sites for extended periods of time while maintaining good thermal contact between the pads and the therapy sites.

### Liposuction Cosmetic Surgery

In the context of liposuction surgery, after the subcutaneous fat has been removed by a long thin suction rod, the skin must reattach to the connective tissue below the dermis layers. Immediately after the surgery the patient is placed in an elastic body girdle that applies uniform and constant pressure to the skin so that the skin smoothly re-attaches to the underlying tissue.

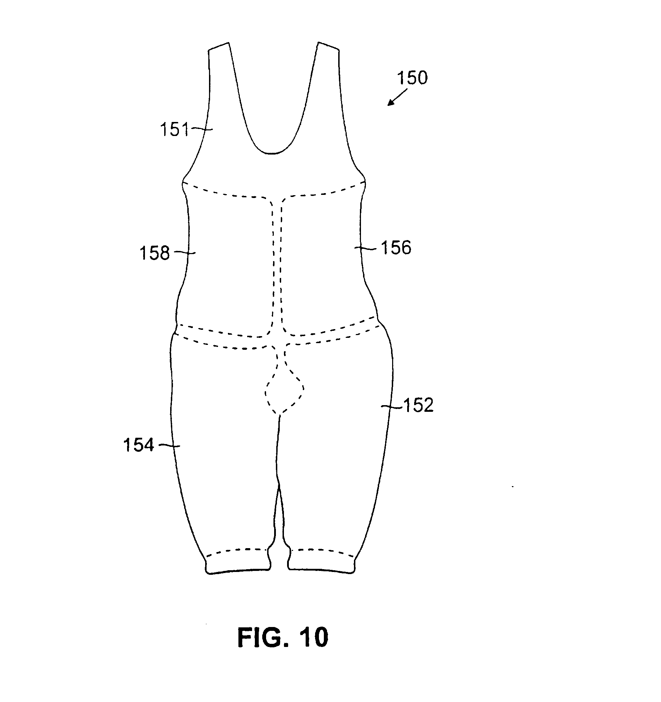

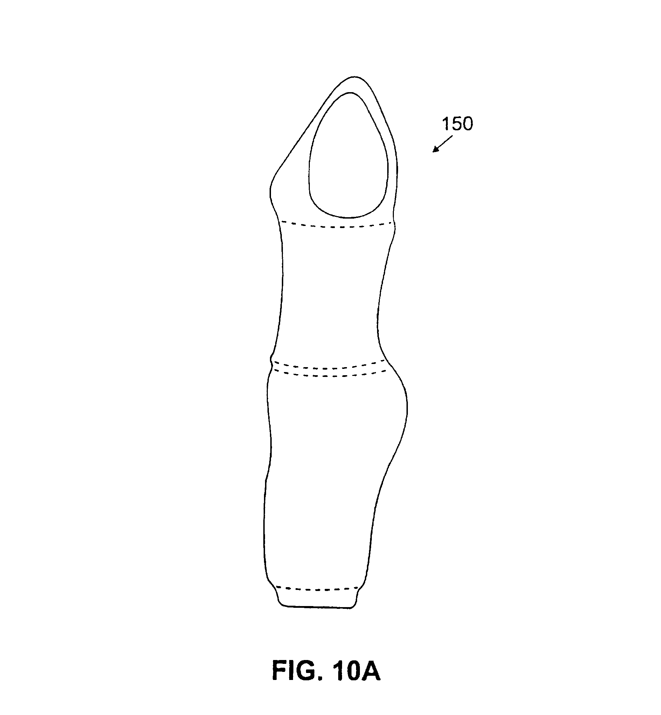

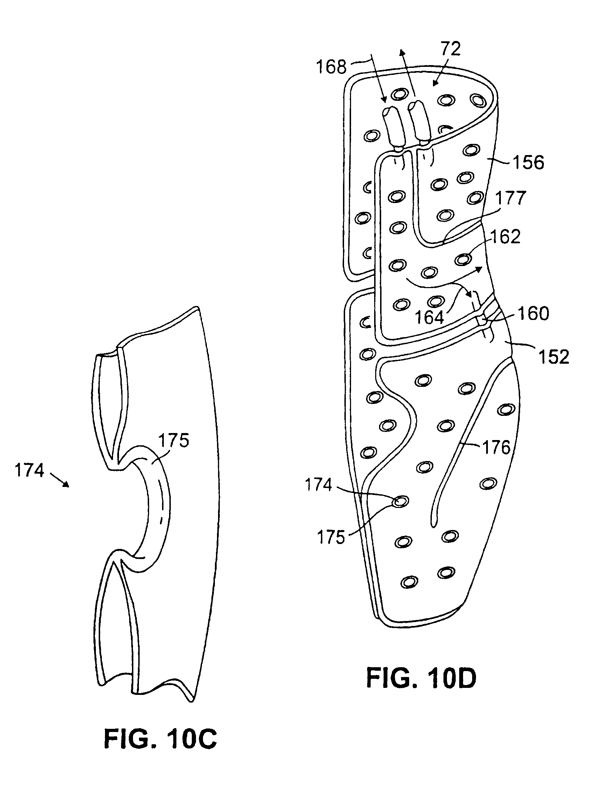

Referring to Figs. 10-10C, a thermal therapy device 150 provides relief for swelling that results from liposuction surgery. Device 150 includes a retainer 151 that incorporates up to four pre-positioned thermal pads 152-158 and is constructed to be worn over the aforementioned body girdle. There is adequate thermal conductivity through the body girdle to achieve adequate swelling reduction without requiring direct contact between the thermal pads and the skin. Retainer 151 is made from a thin, elastic material (e.g., LYCRA® (spandex polyurethane elastomeric fiber)) and provides sufficient compression to "form-fit" the integrated thermal pads against areas likely to swell as a result of surgery. The four thermal pads shown in Fig. 10 are positioned and held in place by form-fitted pockets sewn into the retainer. Each of thermal pads 152-158 is about 18 inches wide and about 22 inches long. Each of the thermal pads can be readily removed and replaced. The thermal therapy device is crotchless so that the device can be used for extended periods of time.

Referring to Fig. 10B, the thermal pads for the thighs and buttocks (152, 154) are coupled to the thermal pads for the upper body (156, 158) by one or more internal pad-to-pad connections 160 which provide a fluid flow configuration that reduces the temperature differential across the area. A single quick-disconnect connector 172, located away from the surgery area, is used to attach the thermal pads to a portable reservoir 16 (Fig. 1) to provide temperature-controlled thermal therapy. The temperature-controlled fluid flows from the front to the back of the patient, as shown by arrow 162, and from pad 156 to pad 152, as shown by arrow 164 (Fig. 10B). The internal pad-to-pad connections 160 reduce the connective tubing requirements. The thermal pads 154, 158, are designed for the right side of the body, the thermal pads 152, 156 are designed for the left side of the body and are mirror images of thermal pads 152, 158, respectively; only two thermal pad molds are therefore needed.

The thermal pads are constructed and arranged to accommodate expected variations in thermal conductivity over the thermal pad area resulting from, e.g., nonuniform application of pressure on the thermal pads. E.g., a patient is often sitting or laying down, which increases the pressure and thus increases the thermal conductivity from the thermal pads to the buttocks or back of the patient. The effects of such thermal conductivity variations are reduced by first flowing the temperature-controlled fluid in those areas (e.g., the regions of the thermal pads corresponding to the front of the patient) that are expected to be under less pressure during use, as indicated by fluid flow arrow 168, and afterwards flowing the temperature-controlled fluid in those areas that are expected to be under higher pressure (e.g., the regions of the thermal pads corresponding to the back of the patient). Alternatively, flow of temperature-controlled fluid is restricted in regions of the thermal pads that are expected to have a higher thermal conductivity path to the therapy sites relative to other regions of the thermal pads (e.g., by increasing the density of dimple welds in regions expected to have a higher thermal conductivity path to therapy sites).

Each of the thermal pads 152-158, includes a plurality of vents 174 that provide air access to the underlying skin. As shown in Fig. 10C, vents 174 are defined by the circumferential edges 175 of the dimple welds. The dimple welds and flow barrier welds 176, 177 control the flow of fluid through the thermal pad and control pad expansion.

### Core Temperature Reduction

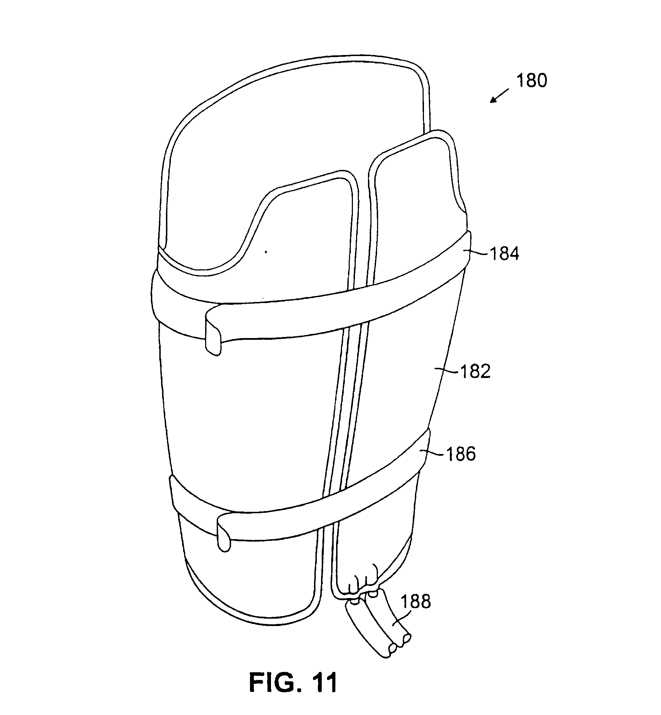

Core temperature control can be used, e.g., to treat excessive fever temperature and control intercranial pressure resulting from a head injury. Referring to Fig. 11, a thermal therapy device 180 includes one or more thermal pads 182 designed in a similar fashion to the thermal therapy device described above in connection with Figs 10-10C.

Thermal therapy device 180 is constructed as a vest and to provide access for external IV leads to one or more of the patient's arms, and for access heart sensors to the patient's chest. With fastener straps 184, 186 unfastened, the vest can be slid under a patient who is laying down, wrapped around the upper body, and then fastened to achieve good thermal contact between one or more thermal pads 182 and the upper torso of the patient. Connection to a portable reservoir 16 (Fig. 1) is done via a quick-disconnect 188.

### Facial Laser Surgery

In laser cosmetic surgery, a patient has the outermost dermis layer ablated or vaporized by the application of a very short, high energy infrared (IR) laser in a controlled pattern. The surgery results in a new, wrinkle-free surface to be formed during the healing process.

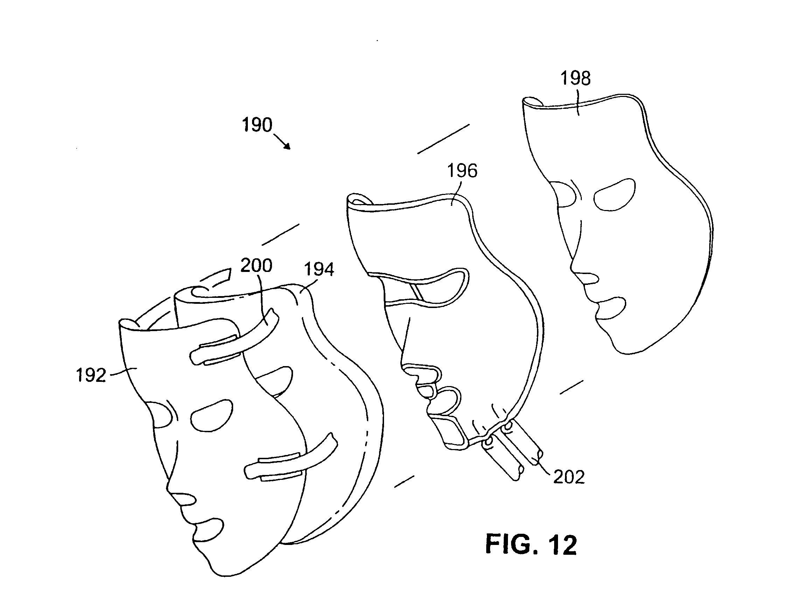

Referring to Fig 12, a thermal therapy device 190 can be used to apply thermal therapy to a person's face, which is usually very sensitive after laser cosmetic surgery. Thermal therapy device 190 includes a three-layer, or optionally four-layer, mask which allows the simultaneous application of topical medication and temperature-controlled thermal therapy to the affected facial areas. An outer layer 192 includes a rigid plastic or aluminum template that is shaped to substantially conform to the contours of a patient's face; an optional intermediate layer 194 is formed from highly compliant foam material shaped to the contours of the patient's face; a thermal pad 198 conforms to the contours of the patient's face; and an inner layer 198 includes highly compliant material with a high thermal conductivity. in one embodiment, layer 198 is an absorbent sponge layer that can be saturated with water to increase its thermal conductivity, and can be used to retain topical medication; the sponge layer is treated with a hygroscopic agent (e.g., glycol) to reduce drying and is impregnated with an antibacterial agents (e.g., iodine) to prevent infection. In another embodiment, layer 198 is formed from a silicon-filled thin-walled plastic container with a thin absorbent sponge layer for contacting the patient's skin. The absorbent sponge layer prevents the skin from scabbing over. The sponge layer is held in place by, e.g., VELCRO® strips, so that it can be easily removed and replaced; the remaining layers are held together by, e.g., VELCRO® strips or double-sided adhesive tape.

An elastic strap 200, causes rigid outer layer 192 to compress the compliant layers 194, 196 against the patient's face. This structure results in good mechanical conformity of the inner layer 198 against the skin with good thermal contact between thermal pad 196 and the skin without requiring use of significant pressure, which would cause the patient pain. The thermal therapy device connects to a portable reservoir 16 (Fig. 1) by a quick-disconnect 202.

### Facial Skin Treatment

In one thermal therapy scheme, thermal therapy device 190 (Fig. 12) is connected to one of the reservoir/pump embodiments described above along with an in-line flow heater coupled to the thermal pad circulation loop. According to this treatment scheme, the sponge layer of the thermal therapy device is impregnated with one or more skin treating ointments, such as vitamin A, vitamin E and thermally activated ointments, and the thermal therapy device is programmed to provide a preselected schedule of thermal treatment. For example, according to one temperature schedule, the thermal pad is initially heated to 102-106° F for approximately 10-15 minutes; this increases skin porosity aiding migration of large molecules (e.g., vitamins A and E) through the dermal layers. After this period, the thermal pad is gradually cooled to a temperature of 40-45° F and held at this temperature for approximately 10 minutes. Finally, the thermal pad is gradually heated to room temperature, at which point the thermal facial skin treatment is complete.

### Dental Oral Surgery

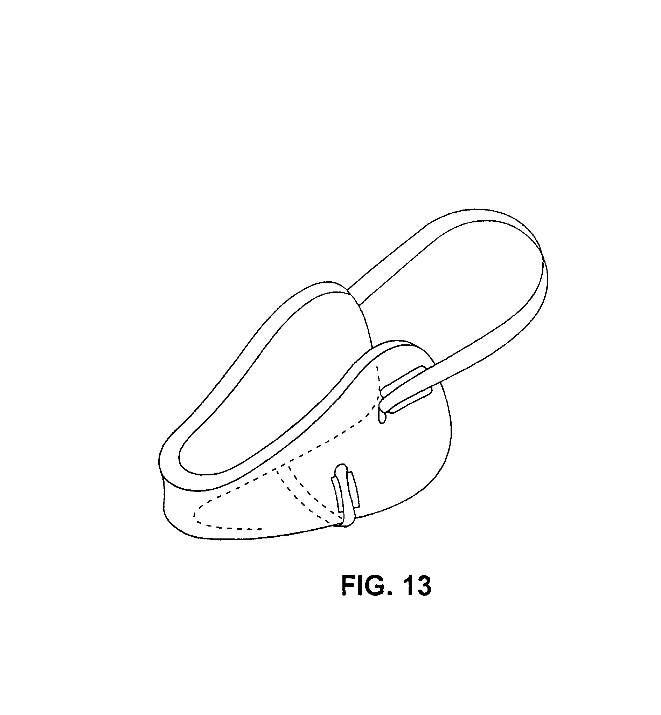

Referring to Fig. 13, a thermal therapy device 210 is constructed to apply temperature-controlled thermal therapy to the upper and lower jaw area and includes the four layers described above in connection with the embodiment of Fig. 12. This device can be used, e.g., in wisdom-tooth extraction and in other forms of oral surgery.

Still other embodiments are within the scope of the claims.

### Hand-Held Control

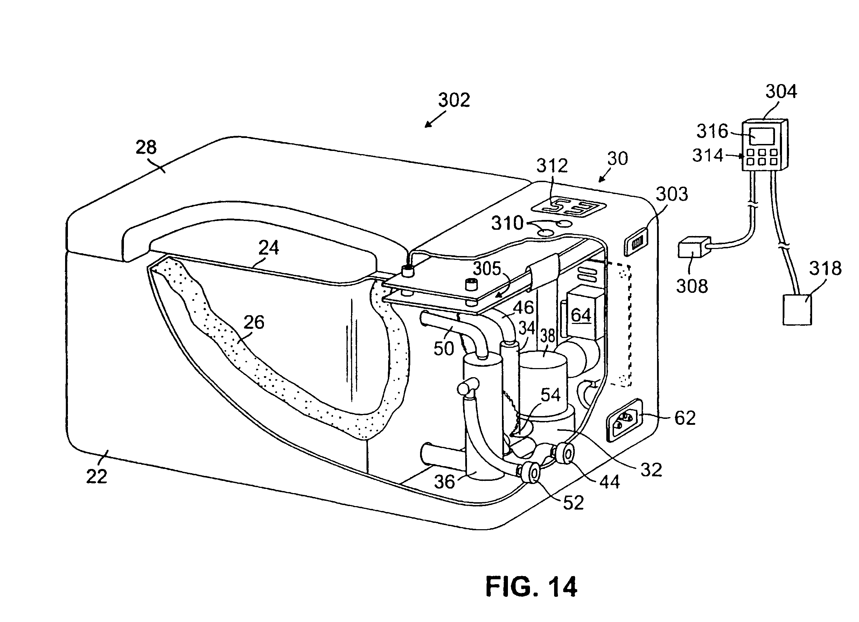

Referring to Fig. 14, in another embodiment, a thermal therapy device 300 includes a portable reservoir 302, which is similar in construction to portable reservoir 16 (Fig. 2), and a hand-held controller 304; hand-held controller 304 can also be used with any of the embodiments of Figs. 7, 8, and 9-9B.

Controller 304 connects to an input 303 (e.g., a RG-11 phone plug adaptor) of thermostat control electronics 305 by a mating connector 308. Input keys 310 are coupled to control electronics 305 and are used to set the thermal therapy temperature to a desired set-point value, which is displayed on a display 312. Thermostat control electronics 305 controls the heat exchanger 31 so that the temperature of the fluid delivered to the thermal pad (not shown) achieves the selected set-point value. Temperature-controlled fluid is delivered to and received from a thermal pad through outlet 44 and inlet 52, respectively.

Hand-held controller 304 incorporates a non-volatile electronic memory for storing, recalling and implementing one or more preprogrammed or user-defined therapy temperature time profiles (e.g., see Fig. 6); hand-held controller 304 also allows for manual temperature control. Input keys 314 are used to program the desired temperature profile into controller memory. The monitored temperature is shown on a digital display 316. Display 316 may also indicate the amount of therapy time remaining. Based on the therapy temperature measured by thermistors 54, controller 304 produces a visual or an audible alarm signal when the cold or heat source in the reservoir is exhausted and the desired therapy temperature cannot be maintained within preset tolerances, or if the unit detects a restricted flow in the circulation system.

The hand-held controller also accepts temperature measurements from a thermometer 318 (e.g., a rectal thermometer). This allows the device to accurately adjust the core temperature of a patient to a desired set-point with feedback control. If hand-held controller 304 is not present, controller 305 defaults to the internal closed loop temperature control provided by thermostat control electronics 305. If hand-held controller 304 is plugged in, it overrides the internal control provided by electronics 305, thus providing enhanced control of the thermal therapy procedure.

Still other embodiments are within the scope of the claims.

## Claims

1. A thermal therapy device for applying thermal therapy to a therapy site on a mammalian body, said device comprising a thermal pad for applying thermal therapy to the therapy site, said thermal pad comprising a plurality of vents for providing air access through said thermal pad when said thermal pad is held in place at the therapy site.

2. The device of claim 1 wherein said vents are defined through a plurality of welds bonding together opposite layers of said thermal pad at a plurality of spaced apart locations.

3. The device of claim 1 or 2 further comprising:
a recirculating fluid loop comprising a fluid channel defined by said thermal pad;
a pump for circulating fluid through said recirculating fluid loop;
a thermal reservoir;
a heat exchanger coupling said thermal reservoir with said recirculating fluid loop; and
a control mechanism coupled to said heat exchanger for enabling adjustable control of therapy temperature.

4. The device of claim 3 wherein said heat exchanger comprises means for delivering a predetermined volume of fluid from said thermal reservoir into said recirculating fluid loop.

5. The device of claim 3 or 4 wherein said control mechanism is coupled to said pump for enabling adjustable control of fluid pressure in said thermal pad, and said control mechanism is adapted to vary pressure of recirculating fluid within said thermal pad in a manner to apply tactile stimulation to a therapy site by increasing and decreasing fluid pressure in said thermal pad.

6. The device of any one of claims 3 to 5 wherein said heat exchanger comprises a second thermal reservoir.

7. The therapy device of claim 6 wherein said heat exchanger comprises a valve for selectively mixing fluid from said first thermal reservoir with fluid from said second thermal reservoir according to a prescribed mixing ratio and for introducing mixed fluid to said pump for circulation in said recirculating fluid loop.

8. The device of claim 1 further comprising:
a recirculating fluid loop comprising a fluid channel defined by said thermal pad;
a thermal reservoir; and
a heat exchanger coupling said thermal reservoir with said recirculating fluid loop, said heat exchanger being constructed and arranged to selectively mix fluid recirculating in said fluid loop with fluid from said thermal reservoir in an adjustable mixing ratio to achieve the selected therapy temperature at the therapy site.
